# EUROPEAN PATENT APPLICATION

(11) **EP 4 529 932 A1**
(43) Date of publication of application: **02.04.2025**
(21) Application number: 23811266.8
(22) Date of filing: 22.05.2023
(51) Int. Cl.: A61L 9/18, B01D 53/86

(54) **PHOTOCATALYTIC SANITISING DEVICE AND METHOD FOR DECONTAMINATING AND SANITISING**

(30) Priority: 23.05.2022 MX 2022006224
(71) Applicant: Universidad de Guadalajara, 44100 Guadalajara, Jalisco (MX)
(72) Inventor: HERNÁNDEZ DE LEON, José Esteban, Jalisco 44800 (MX); LÓPEZ ÁLVAREZ, Miguel Ángel, Jalisco 44100 (MX); REYNOSO GARCÍA, César Alberto, Jalisco 44430 (MX); MEZA DÍAZ, Guillermo, Jalisco 44540 (MX)
(74) Representative: De Clercq & Partners
(86) International application number: PCT/IB2023/055251
(87) International publication number: WO 2023/228051

(57) **Abstract**

The present invention relates to a photocatalytic sanitizing device capable of circulating/conducting ambient air from its immediate surroundings, introducing it into an internal volume and, subsequently, by means of the generation of hydroxyl radicals (OH) resulting from a photocatalytic process, eliminating/reducing the organic matter present in said introduced air stream when it comes into contact with said hydroxyl radicals formed; additionally, the photocatalytic sanitizing device of the present invention has the ability to arrange certain objects in its internal volume, and, through the aforementioned decontamination process, is capable of decontaminating/eliminating/reducing the amount of organic material present on a plurality of surfaces of a certain object, once the air stream entrains/conducts the hydroxyl radicals formed in the photocatalytic process and comes into contact with said plurality of surfaces of the certain object, wherein, the present invention comprises using improved humidity control conditions and the use of improved photocatalytic components in order to substantially and advantageously increase the efficiency of the photocatalytic process.

## Description

The present invention relates, in general, to a sanitizing/decontaminating device that carries out a photocatalytic process capable of eliminating any organic material, such as viruses and/or bacteria present in the ambient air, once it is conducted/directed to an internal volume of the device, and, additionally, eliminating viruses and bacteria present in a given object, once it is placed in said previously referred to internal volume.

The device of the present invention employs a photocatalytic process to generate hydroxyl radicals (OH) which are capable of destroying/eliminating any organic matter upon coming into contact with it.

### PRIOR ART

Recently, there has been a considerable increase in the need to provide spaces with precise and safe control of the level of contamination present in them; in particular, in spaces where there is some type of health risk derived from activity or the presence of crowds, meetings and, in general, the presence of human beings, thus avoiding and/or reducing the possibilities of disease transmission, mainly, diseases related to the respiratory tract (present in the airways) and/or diseases that can be transmitted by coming into contact with the surfaces of certain objects.

Currently, a wide variety of auxiliary devices are known and used to decontaminate and/or purify the air in a given space in a constant and automated manner. For example, the use of filters, screens and/or grids of different sizes incorporated in points with the presence of air currents is known, the purpose of which is to capture organic matter present in said air currents, thus preventing them from reaching their final destination, that is, the space with contamination control in question. Additionally, filters based on minerals with decontaminating, purifying and detoxifying properties and which are capable of adsorbing organic matter, such as activated carbon, zeolites, clays (montmorillonite, sepiolite, bentonite, etc.), the so-called low-cost adsorbents, mostly derived from organic solid waste, and the like, are known and used.

However, the previously mentioned filter examples do not ensure full capture/adsorption of organic materials within an air stream, resulting in a relevant technical problem, particularly in situations where precise control over the retention of organic matter is required when passing through a given space.

On the other hand, purifying/sanitizing devices are known that, unlike mechanical means, employ physicochemical means to eliminate the amount of organic matter; as an example, a wide variety of purifying devices are known that employ a photocatalytic process; said photocatalytic process is based on generating hydroxyl radicals (OH) by preferably irradiating a photocatalytic element with ultraviolet light, where said radicals are strong oxidizing agents capable of, when coming into contact with organic matter, breaking the bonds of nucleic acids causing their destruction/disintegration, thus offering a more efficient decontamination process compared to the previously mentioned mechanical processes.

Typically, photocatalytic elements are semiconductor materials capable of accelerating the rate of oxidation reactions in the presence of a stimulus, which is generally through ultraviolet light radiation (due to the wavelength). A widely used photocatalytic element is titanium dioxide (TiO2) due to its high photocatalytic capacity and in a large number of sanitizing devices based on photocatalytic processes.

In this regard and by way of example, various devices are known in the state of the art that are assisted by a photocatalytic process to reduce the presence of organic matter, such is the case of the device described in the Chinese utility model publication No. CN204006370U, published on December 10, 2014, which describes an automatic indoor air purification device which, using a fan, propels/conducts air from its immediate environment to bring it into contact with the hydroxyl radicals formed by a photocatalytic oxidation unit based on the photocatalysis of silver ions.

On the other hand, US patent 6932947 B2 published on November 13, 2003 discloses a fluid purification and disinfection system, formed with a photocatalytic oxidation device comprising a disinfection core coated by a photocatalytic element arranged in an outer portion thereof; the device of US patent 6932947 B2 carries out a photocatalytic reaction process on the surface of the disinfection core, such that organic matter and contaminants in the fluid can be removed upon contact with the surface of said core. Likewise, international application publication WO2005030370 A1, published on April 7, 2005, and utility model CN205570092U published on September 14, 2016, respectively describe air purifying devices based on a photocatalytic reaction and in which, by the action of a fan, the immediate ambient air comes into contact with the hydroxyl radicals formed. Even devices are known within the prior art, such as the photocatalytic sanitizing device commercially available under the name "sanisol", developed by SAECSA ENERGÍA SOLAR, which combines the effects of the photocatalytic process with mechanical filters to increase the decontamination range of the immediate ambient air.

However, it is known that the photocatalytic process to generate hydroxyl radicals by irradiating, particularly, ultraviolet (UV) light on a photocatalytic element is inefficient or has low efficiency due to the limitation and/or lack of control over the environmental conditions where said process is carried out. The photocatalytic process of the previously mentioned inventions is carried out under uncontrolled ambient conditions, which is a relevant technical problem; a person skilled in the art will know that, under conditions of low humidity and/or constant humidity, the photocatalytic element achieves a low synergy when irradiated by ultraviolet light, thus generating a low and inefficient amount of hydroxyl radicals.

Therefore, technologies have been developed, within the state of the art, that implement a humidity control system, such as, for example, the device of US patent US 11103611 B2, published on July 18, 2019, which is a system to reduce the amount of organic matter using a photocatalytic process and which, by incorporating a humidifier and a water source, the device of US patent 11103611 B2 can control the level of humidity present therein. Similarly, the sanitizing device disclosed in patent application JP2011056155 A, published on March 24, 2011, comprises a container with water and a humidifier with which it can increase/decrease in a controlled manner the humidity present in the device.

However, it is observed that the incorporation of a water source (reservoir/container where the liquid medium is placed) together with an evaporator/humidifier as disclosed in the previously cited documents US 11103611 B2 and JP2011056155 A is impractical; although the water source and the evaporator/humidifier will allow the humidity to be controlled within the respective sanitizing devices, once the liquid contained in said water source is exhausted, the photocatalytic process will again depend on the humidity present and, as previously mentioned, said humidity can fluctuate with respect to time; therefore, the need arises to have a disinfectant/sanitizing system which can carry out efficient humidity control without requiring an auxiliary medium and/or container conditioned to, once exhausted, losing the capacity to control the humidity.

Additionally, as previously mentioned, the use of titanium dioxide (TiO2) is known, commonly used in conjunction with a second photocatalytic component, incorporated into a sanitizing device; however, there are different photocatalytic components with a certain potential that, when interacting with the first photocatalytic component, can generate hydroxyl radicals more efficiently; therefore, the need arises to provide a sanitizing device that comprises a combination of photocatalytic components that offers a more efficient photocatalytic reaction compared to the use of titanium dioxide (TiO2) with water.

Finally, it is observed that the devices currently known in the prior art are intended, and in turn, limited to, decontaminating/sanitizing air in the immediate environment, therefore, the need arises for a device that is additionally capable of decontaminating/sanitizing certain objects, particularly, by eliminating/reducing the amount of organic matter existing on their surface, using and/or relying on the photocatalytic process.

### BRIEF DESCRIPTION OF THE INVENTION

It is therefore an object of the present invention to provide a sanitizing/decontaminating device that carries out a photocatalytic process, capable of eliminating and/or reducing the amount of organic matter through a photocatalytic process; in particular, an object of the present invention is to provide a sanitizing/decontaminating device that carries out a photocatalytic process wherein said photocatalytic process allows to eliminate and/or reduce the amount of organic matter present in an air stream that is introduced/conducted to an internal volume of the photocatalytic sanitizing device.

An additional objective of the present invention is to provide a sanitizing/decontaminating device that carries out a photocatalytic process that allows to eliminate and/or reduce the amount of organic matter present on a plurality of surfaces of a given object, when said object is placed in the internal volume of the sanitizing/decontaminating device based on a photocatalytic process.

Another objective of the present invention is to provide a sanitizing/decontaminating device that carries out a photocatalytic process under controlled humidity conditions, and in particular, without requiring a liquid and/or reservoir/container of liquid intended to be evaporated/condensed to carry out the aforementioned control process.

Likewise, an objective of the present invention is to provide a sanitizing/decontaminating device that carries out a photocatalytic process under controlled humidity conditions, and in particular, where said humidity conditions are controlled by a device commonly known as a heat exchanger and/or, in an even more preferred objective, said humidity conditions are controlled by the photocatalytic components used for the photocatalytic process, through the hygroscopic properties thereof.

An objective of the present invention is, furthermore, to provide a sanitizing/decontaminating device that carries out a photocatalytic process using a combination of photocatalytic components that improves the synergy based on the interaction between both components and, in general, improves the performance and/or efficiency of the photocatalytic process.

Likewise, an objective of the present invention is to provide a sanitizing/decontaminating device that carries out a photocatalytic process that can store/contain within it, particularly in an interior volume, a given object, with the purpose of, once the photocatalytic process is carried out, taking advantage of the air flow introduced/conducted to said interior volume and eliminating any organic matter present on the plurality of surfaces of said object, once the air comes into contact with said surfaces.

In the context of the present invention, "photocatalytic process" should be understood as a physical-chemical process based on the generation of hydroxyl radicals (OH) by irradiating, preferably, with ultraviolet light and/or any other source of radiation emission with an appropriate wavelength a photocatalytic element, wherein said radicals are strong oxidizing agents capable of, when coming into contact with organic matter, breaking the bonds of nucleic acids causing their destruction/disintegration, which, an expert in the art will understand that it is a known and widely used process at present and in no way defines the scope of the present invention; on the contrary, the present invention carries out an improved photocatalytic process based on the selection of photocatalytic components, ratio/proportion between components, operating conditions where the photocatalytic process is carried out, among others, as will be described in greater detail in the present application.

On the other hand, by "photocatalytic sanitizing device" it should be understood that the present invention is intended to be incorporated in such a way that, in its preferred embodiment, as will be described later in this application, it can be used to sanitize/decontaminate the immediate ambient air that is constantly recirculated by the invention and also, to sanitize/decontaminate a specific object that is placed inside it; therefore, the invention should not be considered as limited to a device as shown in the Figures shown below, either in shape and/or dimensions, since the device can be designed in any shape and size in order to increase, for example, the amount of air that can be decontaminated in a given time and/or the amount and/or size of objects placed inside it that can be decontaminated in a single cycle.

### BRIEF DESCRIPTION OF THE FIGURES

[Fig. 1] Figure 1 shows an isometric view of the photocatalytic sanitizing device according to one embodiment of the present invention.
[Fig.2] Figure 2 is a front view of the photocatalytic sanitizing device according to an embodiment of the present invention.
[Fig.3] Figure 3 is a side view of the photocatalytic sanitizing device according to an embodiment of the present invention.
[Fig.4] Figure 4 illustrates a rear view of the photocatalytic sanitizing device according to an embodiment of the present invention.
[Fig. 5] Figure 5 shows a rear view of the photocatalytic sanitizing device according to an embodiment of the present invention, where a portion of the outer structure has been removed, and the internal components of the sanitizing device can be observed.
[Fig.6] Figure 6 is a front, top and side perspective view of the photocatalytic sanitizing device according to a further embodiment of the present invention.
[Fig.7] Figure 7 shows a schematic graph showing the Fourier transform inverse infrared (FTIR) spectrum of TiO2-ZnO analyzed from 500 to 4000 cm-1, where the X axis represents the wavelength, while the Y axis represents the transmittance and where the signals on the X axis are associated with the vibrational modes of the different chemical species present in a material.
[Fig.8] Figure 8 is a schematic graph of the EPR spectrum of TiO2 when irradiated with UV light (λ = 200 nm) for different time intervals, where two intense signals are observed at approximately 334 and 338 mT, which are attributed to unpaired electrons contained in hydroxyl radicals (OH-).
[Fig.9] Figure 9 illustrates a Petri dish placed at the air outlet of the photocatalytic sanitizing device of the present invention in an inverted manner with respect to the surface of the device, showing the preparation position for the analysis carried out during the experimental phase.
[Fig.10] Figure 10 shows a set of Petri dishes, arranged on the top for the air inlet and on the bottom for the air outlet of the photocatalytic sanitizing device of the present invention, for the analysis carried out during the experimental phase.

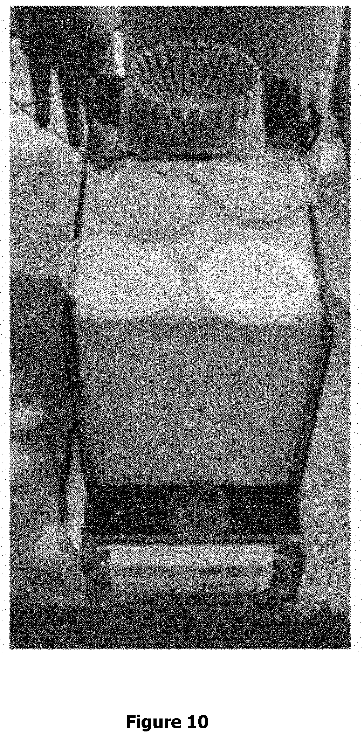

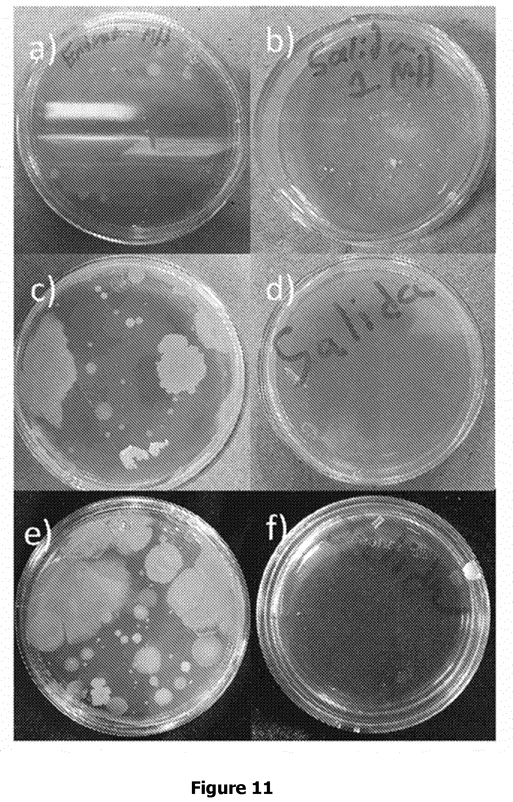

[Fig.11] Figure 11 shows the Petri dishes of Figure 10, after the experimentation, and after an incubation period, where a) and b) are the results of test 1 of control of the air inlet and outlet respectively; c) is the result of the air inlet in test 2; d) is the result for the air outlet in test 2; e) and f) are results in test 3 for air inlet and outlet respectively and where the difference in culture generated in the control Petri dishes (greater presence) and the Petri dishes that came into contact with air sanitized based on the photocatalytic process of the present invention can be observed.

### DETAILED DESCRIPTION OF THE INVENTION

Some aspects of the present invention will now be described in more detail with further reference to the accompanying drawings in which some, but not all, of the advantages of the present invention are shown. Indeed, various embodiments of the invention may be expressed in many different ways and should not be construed as limited to the embodiments described herein; rather, these exemplary embodiments are provided so that this invention will be thorough and complete, and will fully convey the scope of the invention to those skilled in the art. For example, unless otherwise stated, something described as first, second, or similar should not be interpreted as a particular order. As used in the description and appended claims, the singular forms "a", "an", "the" include plural referents unless the context clearly indicates otherwise.

The different aspects of the present invention relate to a photocatalytic sanitizing device that employs a photocatalytic process, capable of effectively eliminating/reducing organic matter from a given space.

More specifically, the present invention relates to a photocatalytic sanitizing device capable of circulating/conducting ambient air from its immediate surroundings, introducing it into an internal volume (11) and, subsequently, by generating hydroxyl radicals (OH) resulting from the photocatalytic process, eliminating/reducing the organic matter present in said introduced air stream when it comes into contact with said hydroxyl radicals formed. Additionally, the photocatalytic sanitizing device of the present invention also has the ability to arrange certain objects in its internal volume (11), and, through the previously mentioned decontamination process, is capable of decontaminating/eliminating/reducing the amount of organic material present on a plurality of surfaces of a certain object, once the air stream drags/conducts the hydroxyl radicals formed in the photocatalytic process and comes into contact with said plurality of surfaces of the certain object, wherein the present invention comprises using improved humidity control conditions and the use of improved photocatalytic components in order to substantially and advantageously increase the efficiency of the photocatalytic process.

In one embodiment, the photocatalytic sanitizing device (1) of the present invention comprises the following main parts:
i) an outer shell (10), which in a preferred embodiment, allows the different components of the photocatalytic sanitizing device of the present invention to be stored and protected inside.

In a preferred embodiment, the outer shell (10) may be made of any suitable material, for example, it may be made of a metallic material, such as and not limited to iron, carbon steel, stainless steel, aluminum, alloys, combinations thereof and/or the like, and/or it may be made of a non-metallic material, such as and not limited to plastics such as PET or PETE (polyethylene terephthalate), HDPE (high density polyethylene), PVC (polyvinyl chloride), LDPE (low density polyethylene), PP (polypropylene), PS (polystyrene), combinations thereof and/or the like.

In a further example, the outer shell (10) may be made of an acrylic type material, in order to provide a shell of reduced weight compared to the use of metallic materials; in a preferred embodiment, an additional treatment may be applied to the material of the outer shell (10); for example, in the embodiment where said shell (10) is made of an acrylic, preferably the acrylic is treated in order to achieve an opaque color or screen, and thereby, dissipate/reduce the amount of radiation exiting from the interior of the photocatalytic sanitizing device (1) of the present invention; as will be described later, the photocatalytic sanitizing device (1) of the present invention comprises a radiation emitter (61) and said treatment to the outer shell (10), i.e. the darkening or opaque color, prevents unwanted exposure to the emitted radiation by a user near the present invention.

In further embodiments, said outer shell (10) may comprise other additional treatments, for example, that improve certain properties; for example, in embodiments where the outer shell (10) is made of a metallic material, a coating may be applied in order to increase resistance to corrosion and/or damage inherent to the weather. One skilled in the art will recognize that such additional treatments are not limited to those set forth herein and that any treatment currently known that promotes certain properties and/or reduces undesired exposure resulting from the components of the present invention may be applied.

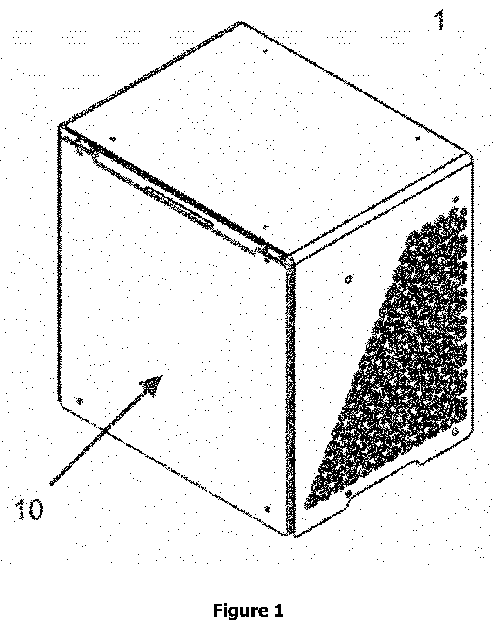

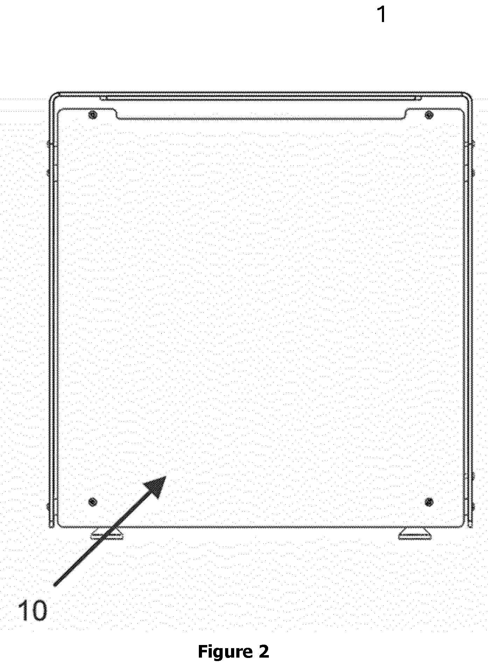

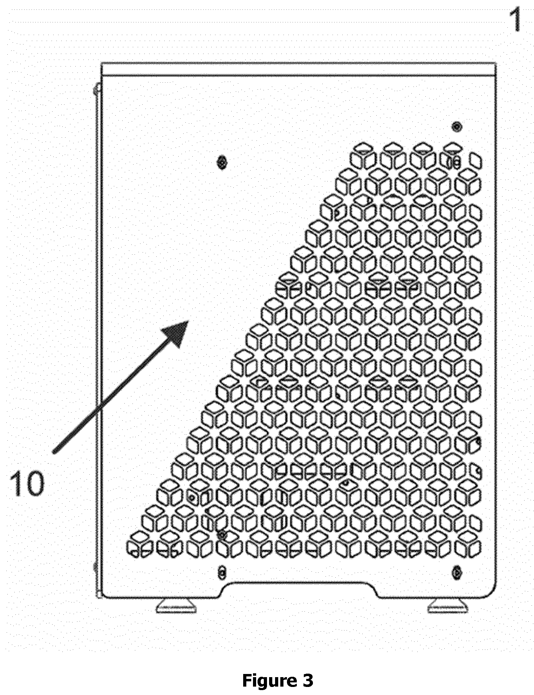

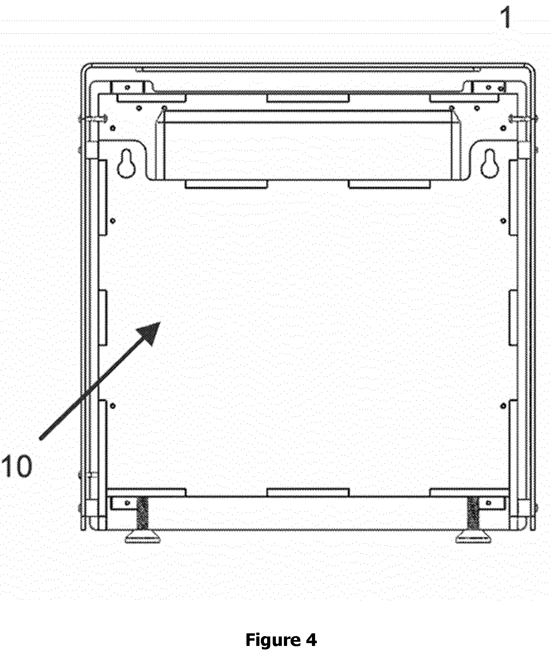

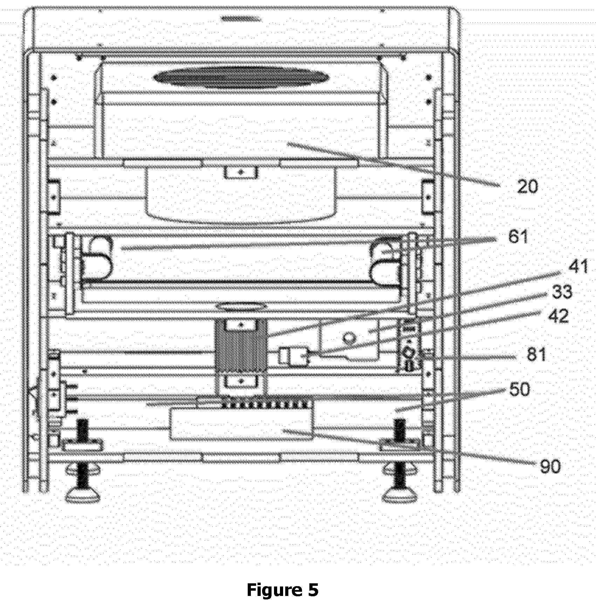

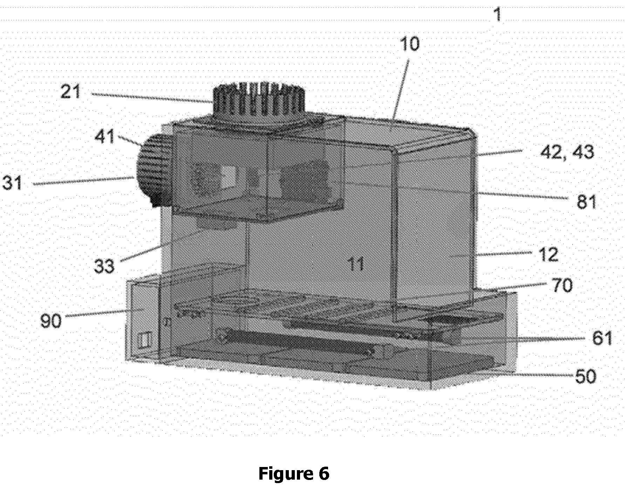

Additionally, the outer shell (10) may comprise any suitable shape, for example, it may form any geometrical body, such as cylinders, prisms, complex and/or irregular shapes and/or combinations of shapes; see, for example, Figures 1 to 5 where an outer shell substantially in the shape of a rectangular prism is shown, which may be an exemplary embodiment of the shell (10) as well as of the photocatalytic sanitizing device (1) and, on the other hand, see Figure 6, where a shell with a shape, size and dimensions totally different from those of the exemplary embodiment of Figures 1 to 5 is illustrated. In this sense, it should be noted that any combination of shapes and dimensions can be applied in a plurality of examples of embodiments without departing from the teachings and scope of the present invention, so that it should not be interpreted that the present invention is limited, in any way, to the shapes, layouts, provisions, arrangements and dimensions shown in the Figures that accompany this description, said Figures being solely illustrative and intended to clearly exemplify the invention that seeks to be protected in this application.

The outer shell (10) may comprise any appropriate dimensions, constituting, independently of the shape adopted, an internal volume (11) and therefore, said dimensions are considered to be directly dependent and/or depend exclusively on the size of the internal volume (11) that is to be provided. Said internal volume (11) can have any volume depending on the dimensions of the photocatalytic sanitizing device (1); by way of non-limiting example, the photocatalytic sanitizing device (1) of the present invention can comprise such dimensions that it generates an internal volume (11) ranging from 50 cm³ to "n" cm³ or from 25 cm³ to "n" cm³ or from 5 cm³ to "n" cm³; in this sense, it should be understood that the present invention can be constituted in any desired shape and/or dimension, thus generating a determined internal volume (11), for example, based on the needs and/or examples of applications/use. As will be described in greater detail in the present application, the photocatalytic sanitizing device (1) is configured to arrange therein a certain object (not shown) to decontaminate/remove any organic material from the plurality of its surfaces based on the photocatalytic process; by way of non-limiting example, the photocatalytic sanitizing device (1) of the present invention may comprise such dimensions and shapes that generate an internal volume (11) sufficient to introduce a relatively small object, that is, whose volume is, for example, 5 cm³, 10 cm³, 15 cm³, 20 cm³, 50 cm³, 100 cm³ or greater; on the contrary, the photocatalytic sanitizing device (1) of the present invention may comprise dimensions and shapes that constitute an internal volume (11) sufficient to introduce a relatively large object, that is, whose volume is, for example, 0.5 m³, 1 m³, 2 m³, 5 m³, 10 m³, 20 m³ or greater.

The interior volume (11) is configured to contain a given object therein; additionally, the interior volume (11) can be filled, completely or partially, by ambient air, which is conducted/drawn by an inlet/intake assembly (20), which will be described in greater detail below.
In a preferred embodiment, the outer shell (10) further comprises an openable gate (12), and which, in an open configuration, allows a user to place a given object on a decontamination pad (70) within the inner volume (11); the foregoing will be described in greater detail later in this application. Once the openable gate (12) is in a closed configuration, it ensures the tightness and airtightness of the internal volume (11) of the photocatalytic sanitizing device (1), such that the air contained within the internal volume (11) can only be expelled or conducted to the outside of the photocatalytic sanitizing device (1) through an outlet (32) in an outlet/expulsion assembly (30), which will be described in greater detail below;
ii) an inlet/intake assembly (20), which in a preferred embodiment, comprises a fan (21) capable of drawing a certain amount of ambient air from the immediate environment, and directing it to the interior volume (11) formed by the outer shell (10) as previously described.

The inlet/intake assembly (20) is capable of introducing ambient air from the outer portion of the photocatalytic sanitizing device (1) of the present invention and conducting it to the inner volume (11) at an appropriate volumetric rate/flow; in this sense, the inlet/intake assembly (20) and, in particular, the previously referred to fan (21), can be of any appropriate dimension, shape and blade profile, and being capable of displacing/dragging a volumetric flow ranging from 0.5 kg/m³ and up to "n" kg/m³.
In this regard, the fan (21) of the inlet/intake assembly (20) may be made of any suitable material, such as and not limited to a metallic material, such as and not limited to iron, carbon steel, stainless steel, aluminum, alloys, combinations thereof and/or the like, and/or it may be made of a non-metallic material, such as and not limited to PET or PETE type plastics (polyethylene terephthalate, HDPE (high density polyethylene), PVC (polyvinyl chloride), LDPE (low density polyethylene), PP (polypropylene), PS (polystyrene), combinations thereof and/or the like;
iii) an outlet/expulsion assembly (30), which in a preferred embodiment, comprises a mechanical filter (31) in order to offer a subsequent filtering step after sanitization by means of the photocatalytic reaction process, as will be described in greater detail below.

The outlet/expulsion assembly (30) can release the air contained in the interior volume (11) formed by the outer shell (10), once said air has come into contact with the free radicals formed by the photocatalytic process and, therefore, has suffered a partial and/or total reduction of organic matter with respect to the amount of the same present when entering the photocatalytic sanitizing device (1).

The outlet assembly (30) may comprise, in an additional embodiment, an outlet (32), such that, once the previously mentioned decontamination process has finished, said outlet (32) can switch from a closed state to an open state and thereby release the already decontaminated air.

In a further embodiment, the outlet assembly (30) may not comprise any opening or mechanical closing/opening means, and may, on the contrary, have a filter that offers a certain resistance to the exit of the air contained in the interior volume (11). For example, and in a non-limiting manner, the contained air can remain within the internal volume (11) from 1 second to "n" number of seconds; likewise, the contained air can remain within the internal volume (11), circulate through it, and be expelled from it at a certain range and/or exit speed, such that it can be in contact long enough to interact with the hydroxyl radicals and eliminate/remove the organic matter therein.

In a preferred embodiment, the outlet/expulsion assembly (30) may comprise a mechanical filter (31), for example and not limited to a filter formed by activated carbon pellets and/or any other material with inherent decontaminating and/or purifying properties, which, in addition to offering a certain resistance to the passage of air when trying to exit the internal volume (11), may act as an additional decontamination step, removing/withdrawing the organic matter present in the sanitized air (in case there is a remainder) that has been exposed to the photocatalytic process prior to being released/expelled back into the environment.

Additionally, in one embodiment, the outlet/expulsion assembly (30) may further comprise at least one air quality sensor (33), configured to determine and/or check the amount of contaminants/organic matter present and/or remaining in the air contained within the internal volume (11); particularly, the air quality sensor (33) may, once the photocatalytic process is carried out, analyze and determine the amount of contaminants/organic matter.
As will be described later in this application, said air quality sensor (33) is in communication with at least one microcontroller (81) of a control unit and with this, it can be determined if the photocatalytic process is being carried out efficiently; in one embodiment, the air quality sensor (33) can be, without limitation, a GP2Y1014AU dust and/or air quality sensor, a ZH0 laser dust sensor, an HIC192 infrared dust sensor, a QSA2700 dust sensor, a particles per million sensor, combinations thereof and/or the like;
iv) a humidity controller, which in one embodiment of the present invention, may be a heat exchanger (41), which allows a controlled amount of energy, particularly thermal energy, to be transmitted and conducted to the ambient air once it is conducted/drawn by the inlet/intake assembly (20).

The heat exchange element (41) is configured to transmit energy and increase/decrease the temperature of a fraction of the air that is drawn into the internal volume (11), with the intention of condensing a fraction of the humidity present in said air. The above aims to provide the internal volume (11) with a desired humidity, in a controlled manner, thus favoring an efficient or more efficient condition for the photocatalytic process. In this sense, "an efficient or more efficient condition for the photocatalytic process" should be understood as meaning that, as is known in the prior art, the photocatalytic process has a higher performance in terms of efficiency under conditions at a certain constant humidity. Thus, the heat exchange element (41) advantageously uses the humidity present in the ambient air to generate said conditions without requiring a reservoir/container comprising a liquid medium arranged and/or configured to evaporate and/or vaporize.

In one embodiment, the heat exchanger element (41) may be at least one and up to "n" number of heat exchanger components distributed, by way of example and not limitation, in the fan terminal (21) of the inlet/intake assembly (20), and/or distributed in different portions of the internal volume (11) and/or in a portion close to the outlet of the outlet/expulsion assembly (30), combinations thereof and among others.

In a further embodiment, the heat exchange element (41) may be an electrothermal device, such as and not limited to a resistor, a resistor bank/set (Joule effect); even more preferably, the heat exchange element (41) may be a Peltier Plate/Cell, for example and not limited to a 12706 Thermoelectric Peltier Cell Cooler 12v 5A 60W, as well as combinations of the previously referred to electrothermal devices.

Optionally, the Peltier plate/cell of the heat exchange element (41) can advantageously transmit a certain amount of energy and condense a fraction of the humidity present in the air as previously described, and additionally, said fraction of condensed humidity can descend, by gravity, and be contained/absorbed by a retainer/mixer assembly (50), which will be described in greater detail later.

Optionally, the condensed moisture fraction may be retained in said retainer/mixer assembly (50) and advantageously, said liquid medium (resulting from the condensation) may be used for the photocatalytic process, as an amalgamating medium, and/or may be used as an additional means to control the moisture level within the internal volume (11). In this way, advantageously, the heat exchange element (41) can be used to constantly feed the photocatalytic sanitizing device (1) with the required liquid and can advantageously be stored in the retainer/mixer assembly (50).

In a further embodiment, the photocatalytic sanitizing device (1) of the present invention further comprises at least one humidity level sensor (42), as well as at least one temperature sensor (43) incorporated in the internal volume (11) and/or distributed in different portions thereof; said humidity level sensor (42) and said temperature sensor (43) may be respectively connected to a microcontroller (81) of the control unit, which will be described in greater detail below. In this embodiment, the humidity level sensor (42) can detect an inadequate overall and/or average level of humidity within the internal volume (11) and the microcontroller (81) can increase/decrease the voltage/current flowing through the heat exchanger element (41) in order to increase/decrease the amount of energy transmitted to the ambient air entering the photocatalytic sanitizing device (1) of the present invention and, on the other hand, the temperature sensor (43) can detect changes in the temperature of the air contained in the internal volume (11), feedback said information to the microcontroller (81) in order to improve the aforementioned energy transfer efficiency.

In this regard, in one embodiment, the humidity and temperature sensor (42, 43) may be combined/integral sensors such as and not limited to Dht21/am2301, Dht11, Sht30, SHT31-ARP-B temperature and humidity sensors, combinations thereof and/or the like.

In an even more preferred embodiment of the invention, the humidity control within the photocatalytic device (1) described and sought to be protected in the present application, can be carried out in an even more advantageous manner and/or providing improved efficiency of the humidity control, relying on the hygroscopic properties of the photocatalytic components, even without the use of a heat exchanger element (41).

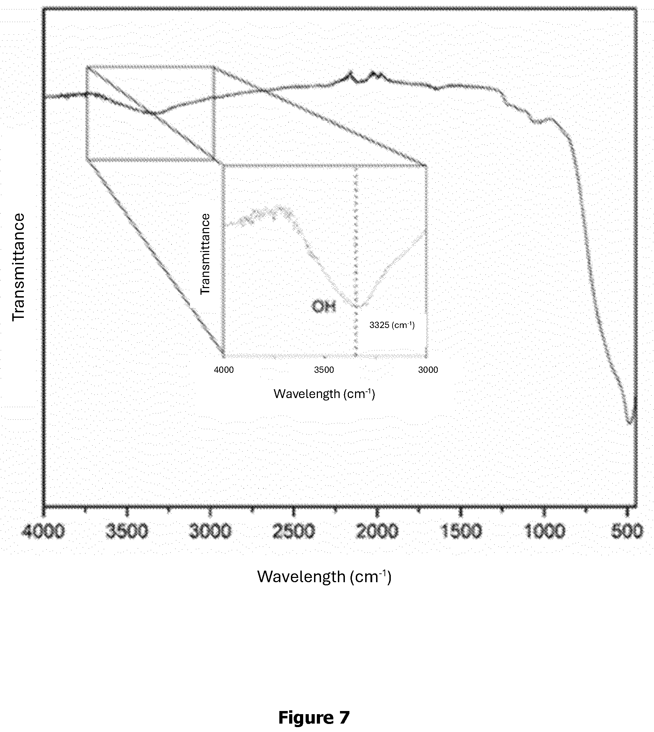

Taking as reference what is illustrated in Figure 7, the applicant has determined and discovered, based on experimentation, that the use of photocatalytic components comprising, in particular, a hygroscopic property allows to efficiently control the humidity present in the internal volume (11) of the photocatalytic sanitizing device (1) and, advantageously, avoiding the use of an additional/auxiliary element such as a heat exchanger (41) as previously described in the present application.

Likewise, the applicant has discovered a set/combination of photocatalytic components, which are titanium dioxide (TiO2) and zinc oxide (ZnO), which are advantageously used to carry out the photocatalytic process and/or reaction, causing a more efficient decontamination/sanitization. In addition, the hygroscopic property observed in both components can be favored, mainly, derived from the existing synergy achieved based on their combination and use as claimed in this application.

A person skilled in the art will know that the hygroscopic property of certain components refers to the capacity present in certain substances/materials that allows them to adsorb/retain/attract and/or contain water, either in the form of vapor and/or liquid from the immediate environment with which they interact; considering the above, it is known that the photocatalytic components used in the present invention, such as, firstly, titanium dioxide (TiO2) is a hydrophilic component, with a low to medium hygroscopic capacity; the same can be said of the second photocatalytic component used in the present invention, that is, zinc oxide (ZnO); however, the combination of both components (TiO2 + ZnO), and derived from the ratio proposed in the present application, triggers an interesting, advantageous and extremely important effect. As will be described in more detail later in this paper, the proposed ratio of the photocatalytic components titanium dioxide (TiO2) and zinc oxide (ZnO) and based on the synergy during the reaction of both components, which is linked, dependent and/or directly resulting from the proposed ratio, further improves the overall hygroscopic property.

Based on Figure 7, where the X axis represents the wavelength, while the Y axis represents the transmittance and where the signals on the X axis are associated with the vibrational modes of the different chemical species present in a material, it is observed that the signal located at approximately 510 cm-1 is related to the TiO2-ZnO junctions. This signal (being the most intense in the spectrum) shows that the main component of this material is titanium dioxide and zinc oxide. On the other hand, the small signals located in the region of 1000 to 1250 cm-1 can be related to vibrational modes of chemical species with CO (Carbon-Oxygen) bonds. The presence of these signals shows that the material can adsorb part of the CO2 (Carbon Dioxide) from the environment and decompose it chemically, forming some types of carbonates; which is why these signals are shown, which are unrelated to the chemical composition of the material. Finally, the signal located in the region of 3000 to 3700 cm-1 (which is amplified in a window inserted within the same figure) and centered at 3325 cm-1 is associated with chemical groups with OH (Oxygen-Hydrogen) bonds. The presence of this signal is attributed to the adsorption of H2O (water) from the environment.

In this way, both photocatalytic components can, based on this improved and/or increased general hygroscopic property, absorb/attract the water (in the form of vapor) present in the ambient air that is introduced into the internal volume (11) of the photocatalytic sanitizing device of the present invention, thus achieving an increase in the level of humidity with which the photocatalytic components interact, advantageously improving the efficiency of the photocatalytic process, thus avoiding, as previously mentioned, the use of a heat exchanger element to carry out said function.

v) a retainer/mixer assembly (50), which, as previously described, is capable of retaining/storing different materials by absorption; in a preferred embodiment, said retainer/mixer assembly (50) is a porous material, or with a certain density of porosities, for example and not limited to, a sponge, foam materials based and/or manufactured from ethylene-vinyl acetate polymers (EVA), polyethylene (PE), combinations thereof and/or the like; in general, the retainer/mixer assembly (50) can be manufactured from a porous material, with the capacity to retain/absorb liquids, granular solids (pulverized and/or in powder form).

In one embodiment, the retainer/mixer assembly (50) is configured to, as previously mentioned herein, retain/absorb a certain amount of condensed liquid, originating from the condensation of a fraction of humidity present in the air within the internal volume (11). In an optional embodiment, the contained liquid may be water, and in one embodiment, said water may be placed directly on the retainer/mixer assembly (50) and may not necessarily be provided by the condensation of a fraction of humidity present in the air within the internal volume (11); said liquid has the purpose of containing and mixing the photocatalytic components (described later in this application) to amalgamate both components (TiO2 and ZnO), advantageously avoiding the formation of lumps, which consequently results in an improvement in the efficiency of the photocatalytic process; in an even more preferred embodiment, the contained liquid is double-distilled water, which, derived from its double demineralization process, whether by double distillation, double demineralization or double reverse osmosis step (and/or other known and/or used processes), is a liquid with a high level of purity, advantageously favoring the efficiency of the photocatalytic process.

In a preferred embodiment, the retainer/mixer assembly (50) can receive both photocatalyst components (TiO2 and ZnO) from the respective storage tanks (62) as will be described in greater detail in this application.

The retainer/mixer assembly (50) can store/retain a certain amount of photocatalytic components, which can be from 0.5g to "n" number of grams and up to 0.5ml, and up to "n" number of milliliters, depending on the type of presentation of both elements.

Furthermore, as will become clearer later, the retainer/mixer assembly (50) may also, in one embodiment, store/retain a given number of photocatalytic components, wherein it may store/retain an equal, greater and/or lesser amount of one photocatalytic component, for example titanium dioxide, relative to an amount of another photocatalytic component, for example zinc oxide.

On the other hand, the retainer/mixer assembly (50) may not be limited to a porous material as previously described; in optional embodiments, the retainer/mixer assembly may contain, in any other form, the photocatalytic components, for example and not limited to, a solid form such as a tablet (not shown) that reacts in the same way as the porous material once it receives and/or is irradiated with UV light (as explained below in this application), and/or in any other form/medium such that it favors a photocatalytic reaction according to the process described in this application.

vi) a photocatalytic/photocatalyst assembly, which in turn is made up of: a radiation emitting source (61) and at least one storage tank for each of the photocatalytic components (62a, 62b). In a preferred embodiment, the radiation emitting source (61) can be any currently known means capable of generating electromagnetic waves, such as and not limited to, germicidal lamps, wide frequency ultraviolet radiation lamps, for example and not limited to from 100 to 200 nm, magnetrons and/or controlled microwave emission means, combinations thereof, and/or the like.

Prior to the start of the photocatalytic process, the respective storage tanks (62a, 62b) of each photocatalytic component release a certain amount of each photocatalytic component respectively, and deposit it in the retainer/mixer assembly (50) as previously mentioned.

In one embodiment, the mixing ratio of the first photocatalytic component and the second photocatalytic component that is released and mixed may be 1:1; in a further embodiment, said ratio may be different than 1:1.

In a preferred embodiment, the applicant has discovered an advantageous mixing ratio between both first and second photocatalytic components that is released and mixed in the retainer/mixing assembly (50); advantageously, said ratio proposed in the present application, firstly increases the synergism between both components, which causes that, once the photocatalytic reaction has started, a larger surface is covered and therefore, a larger surface can be inoculated, also offering greater adherence to the surface on which it is deposited, even in the presence of an air flow, even managing to carry out said photocatalytic reaction in less time and using a smaller amount of resources. The proposed mixing ratio creates energy sublevels smaller than the band gap energy levels, even promoting the photocatalytic reaction to occur at wavelengths shorter than the Ultraviolet.

Advantageously, the applicant has found that a 9:1 ratio of the mixture of both first and second photocatalytic components respectively favors or causes the aforementioned conditions, such that, preferably, the respective storage tanks (62a, 62b) are used and/or caused to release said ratio constantly.

In this sense and in the context of the present invention, the first photocatalytic component is titanium dioxide (TiO2) and the second photocatalytic component is zinc oxide (ZnO); for the sake of clarity, what was previously mentioned about the mixture ratio between both photocatalytic components, in the context of the present invention, should be interpreted that titanium dioxide (TiO2) is in a proportion of 9:1 with respect to zinc oxide (ZnO).

Advantageously, it has been found, as shown by experimental results to be described later in the present description, that the combination of titanium dioxide (TiO2) and zinc oxide (ZnO), referred to in the present application as a mixture ratio, shows that a preferred ratio of 9:1 offers a more efficient photocatalytic process compared to a different ratio, for example, a 1:1, 2:1, 3:1, 4:1, 5:1, and/or 1:2, 1:3, 1:4, 1:5 ratio; however, the present invention may utilize any ratio, for example 1:1, 2:1, 3:1, 4:1, 5:1, 6:1, 7:1, 8:1, 10:1, 20:1, 50:1 or higher, as well as 1:2, 1:3, 1:4, 1:5, 1:6, 1:7, 1:8, 1:9, 1:10, 1:20, 1:50 or higher without departing from the teachings of the present invention. Additionally, the applicant has detected that, based on the use of the previously mentioned 9:1 ratio, any degree of toxicity is considerably and advantageously avoided, thus providing a photocatalytic sanitizing device (1) that can be used in any condition and without any possibility of causing harm to humans, the environment and/or animals.

Optionally, for the photocatalytic process of the present invention other photocatalytic components known within the field to which the invention belongs can be used, however, reiteratively, emphasis is placed on the use of titanium dioxide (TiO2) and zinc oxide (ZnO) in the aforementioned ratio, that is, 9:1 respectively, and which is a novel and innovative ratio, advantageously favors a more efficient, rapid and economical decontamination.

Once the radiation emitting source (61) is turned on, said radiation is supplied directly onto the previously mentioned retainer/mixer assembly (50). Thus, the mixture of photocatalytic components with the advantageous, novel and innovative proportion, described previously, when irradiated, initiates a process of release of hydroxyl radicals (OH), which will spread/distribute once they come into contact with the air within the internal volume (11); in this sense, it is known within the field to which the invention belongs that said hydroxyl radicals have the capacity to decontaminate/remove/reduce and/or eliminate all organic matter present in the air and/or on surfaces, once said radicals come into contact with the organic matter.

In the context of this application, "organic matter" should be understood as any organic form present and/or that may be present in the ambient air, being for example, viruses, bacteria, microorganisms, odors, and the like, and which are mostly potentially harmful to human health.

In a further embodiment, the storage tanks (62a, 62b) previously referred to may be dispensed with and both photocatalytic components may be directly or previously arranged within the retainer/mixer assembly (50), complying with the advantageous mixing ratio 9:1 previously referred to, such that a photocatalytic process may be carried out in the form of batches and/or cycles, limited to the quantity present in the retainer/mixer assembly (50) being exhausted. Likewise, as previously mentioned, given that both photocatalytic components can be arranged in different types of shapes, not limited to porous materials, in said additional embodiment, by dispensing with the storage tanks (62a, 62b), the retainer/mixer assembly (50) can be used and/or arranged in such a way that it can receive the radiation from the radiation emitting source and carry out the photocatalytic process as described throughout this description.

viii) a decontamination base (70), which may be a structure connected and/or attached to or that mechanically interacts with the external shell (10), particularly, in a lower internal portion thereof, and which, in a preferred embodiment, is arranged in the upper part or in a portion above the previously referred to photocatalytic assembly, as seen in the attached Figures.

The decontamination base (70), in a preferred embodiment, is made of the same material as the outer shell (10) and its dimensions are directly related to the shape and dimensions adopted by said shell (10), such that it preferably covers the entire transverse plane, in order to increase its useful surface.

In one embodiment, the decontamination base (70) is configured so that a user can place on its upper surface any given object (which can be introduced depending on the capacity of the internal volume (11) as previously described), such that, during the photocatalytic and decontamination process described previously, that is, during the interaction between the hydroxyl radicals and the air within the internal volume (11), said air drags and directs said radicals so that they come into contact with the plurality of surfaces of the object placed on the base (70), thus achieving that the object is stripped and/or all organic matter present therein is eliminated.

Said decontamination base (70) may comprise, in a preferred embodiment, grooves passed through its surface, so that the hydroxyl radicals formed in the photocatalytic process as previously described can circulate through the base.

In an optional embodiment, the photocatalytic sanitizing device (1) of the present invention may dispense with a decontamination base (70), such that the present invention decontaminates only the air introduced into the interior volume (11), particularly in favor of a more compact and/or smaller photocatalytic sanitizing device (1), compared to the same when it has the previously mentioned decontamination base.

vii) a control unit, which in turn is formed by at least one microcontroller (81) connected to a plurality of sensors in order to control the different components of the present invention; the microcontroller (81), in a preferred embodiment, is also connected to the radiation emitting source (61), whereby it can start and/or conclude a cycle of the photocatalytic process depending on the exposure time of the air/object to the hydroxyl radicals and/or depending on the air quality detected by the air quality sensor (33), as previously described.

Also, according to one embodiment of the present invention, as described herein, the microcontroller (81) is further connected to the humidity controller, particularly in the embodiment where a heat exchanger element (41) is used in order to control the operating time and/or energy supplied to it and thereby control the level of humidity present in the internal volume (11) of the photocatalytic sanitizing device (1).

The microcontroller (81) may be further connected, in accordance with an embodiment of the present invention, to the storage tanks (62a, 62b) of the photocatalytic components, such that said microcontroller (81) can coordinate the respective precise and controlled release of the first and second photocatalytic components and thereby ensure that the previously mentioned novel and innovative mixing ratio (9:1) is met.

In one embodiment, the control unit may comprise at least one and up to "n" number of microcontrollers (81), which may be arranged, interact and/or communicate in a plurality of ways with the different components of the photocatalytic sanitizing device (1) of the present invention. In this context, by "they can be arranged, interact and/or communicate in a plurality of ways" it should be understood that each component comprising the present invention can be connected and interacted with the at least one microcontroller (81) and/or can comprise a microcontroller (81) associated with that single component (as a sub-microcontroller and which subsequently connects/interacts with a master and/or main microcontroller), and/or a general microcontroller can be arranged to selectively connect/interact; in this sense, it should be understood that these variations are not limited to those described herein and that a person skilled in the art could propose/suggest and/or interpret other types of connections/interactions between the plurality of microcontrollers of the control unit and the different components comprising the photocatalytic sanitizing device (1) of the present invention.

In a preferred embodiment, the at least one and/or the plurality of microcontrollers (81) may be any commercially available microcontroller, for example and not limited to AMCC, Altera, Analog Devices., Atmel, Charmed Labs, Cypress MicroSystems, Dallas Semiconductor, ELAN Microelectronics Corp, Freescale Semiconductor, Fujitsu, Holtek, Infineon, Intel, Lattice Semiconductor, Microchip Technology, National Semiconductor, combinations thereof and/or the like.

ix) a power supply (90) capable of feeding the different components that comprise the photocatalytic sanitizing device (1) of the present invention; without any limitation, commercially known direct current and alternating current power supplies (90) with different types of technical specifications can be used; in a preferred embodiment, the power supply (90) can be at least one and up to "n" number of power supplies; likewise, the at least one and/or the plurality of power supplies (90) can be, by way of non-limiting example, a 12v, 10a 120w, 110vac 220vac switched power supply, a 12v or 5v mini switched power supply, combinations thereof and/or similar.

In this regard, an expert will understand that the power supply (90) is not limited to the previously mentioned types of power supplies, and that any related variant, whose function is to feed and/or energize electronic components, such as those previously referred to, comprising the photocatalytic sanitizing device of the present invention would be contemplated within the scope of the present invention; likewise, an expert will understand that the one and/or the plurality of power supplies (90) can be arranged in any portion inside/outside the photocatalytic sanitizing device (1) and not necessarily in the manner as observed in the attached Figures.

The present invention, as described throughout this document, also contemplates the implementation of a method to decontaminate/sanitize/reduce the amount of organic matter in the ambient air near the photocatalytic sanitizing device, relying particularly on the photocatalytic process and, mainly, on the innovative and novel mixing ratio of photocatalytic components (9:1).

Thus, in accordance with any of the described embodiments of the photocatalytic sanitizing device (1) of the present invention, once said device is energized and/or switches to an operating state, the method of the present invention comprises:
i) suctioning contaminated air from the immediate environment, i.e., close to the photocatalytic sanitizing device (1) of the present invention, which may be air within a closed space such as a room and/or a certain area, and conducting/directing it into the interior of the photocatalytic sanitizing device (1) through the operation of a fan (21); in particular, the ambient air is conducted from the inlet/intake assembly (20) and directed and contained in the internal volume (11) of the photocatalytic sanitizing device (1);
(ii) ensuring a controlled humidity level, using, according to the embodiment employed, the heat exchanger element (41) or relying on the improved general hygroscopic property derived from the synergy of the photocatalytic components and the innovative and novel mixing ratio thereof; in this regard, the humidity level must be controlled in order to ensure sufficient humidity to increase the efficiency of the photocatalytic reaction and thus increase the sanitization efficiency;
iii) activating the radiation emitting source (61) and, consequently, irradiating and/or supplying/directing the radiation emitted by it to the retainer/mixer assembly (50), thus starting the photocatalytic reaction process; in this regard, the photocatalytic reaction process, as referred to throughout this description, derived from the radiation of the radiation emitting source (61) on the retainer/mixer assembly (50), which contains the photocatalytic components in a mixture according to the innovative and novel mixing ratio proposed in this application, hydroxyl radicals (OH) are generated, which will be released and spread (based on particle dynamics) within the internal volume (11) of the photocatalytic sanitizing device (1);
iv) removing any organic matter present in the internal volume (11) of the photocatalytic sanitizing device (1) of the present invention, once the hydroxyl radicals (OH), generated during the enhanced photocatalytic process come into contact therewith; wherein, in this step, the organic matter present in the internal volume (11) of the photocatalytic sanitizing device (1) is reduced, which may be contaminated air introduced in step i) and, in addition, it may be the organic matter present on a plurality of surfaces of a given object that has been placed in the internal volume (11), particularly, deposited on the decontamination base (70); although the contaminated air within the internal volume (11) and which comes into contact with the hydroxyl radicals formed in step iii) is in continuous movement, said air allows the hydroxyl radicals to be conducted/directed until they come into contact with the plurality of surfaces of the determined object arranged in the internal volume (11), thus achieving that the contaminants in the determined object are reduced/eliminated;
v) evaluating the quality of the air within the internal volume (11) of the photocatalytic sanitizing device (1) of the present invention, using the plurality of sensors and after the air within the internal volume (11) has been exposed to the hydroxyl radicals resulting from step iii); such that, if the plurality of sensors determine that the quality of the air contained in the internal volume (11), it will be determined that the efficiency of the photocatalytic process is not the desired and/or adequate one and, consequently, the radiation emitting source (61) will continue to be on, causing the photocatalytic reaction to continue, and hydroxyl radicals to be generated uninterruptedly; where the plurality of sensors can track and/or determine the quality of the air contained in the internal volume (11) in different periods of time.
vi) expelling the air (now sanitized/decontaminated) from the internal volume (11) and directing it back to the immediate environment, once in step v) it is determined that the air quality is at a high level, i.e. that the amount of contaminants/organic matter is low and/or null; the outlet/expulsion assembly (30), comprising an outlet (32), opens said outlet so that the air (already sanitized/decontaminated) is directed out of the internal volume (11) towards the environment; where, prior to coming into contact with the environment, the air (already sanitized/decontaminated) passes through a mechanical filter (31) in order to retain any remaining contaminants.

At this point, once the air (sanitized/decontaminated from step vi) is expelled from the internal volume (11) of the photocatalytic sanitizing device (1) of the present invention, steps i) to vi) previously referred to are repeated again, uninterruptedly and/or cyclically such that the photocatalytic sanitizing device can gradually eliminate organic matter, taking fractions of contaminated air from the immediate environment and until the same (contaminated air), in a certain operating time, is completely decontaminated, that is, there are no remnants of organic matter in the room/area of interest.

### EXAMPLES

The experimental processes and the results obtained from them will be described below, which allow supporting and/or confirming the improved, advantageous, novel and innovative efficiency of the present invention.

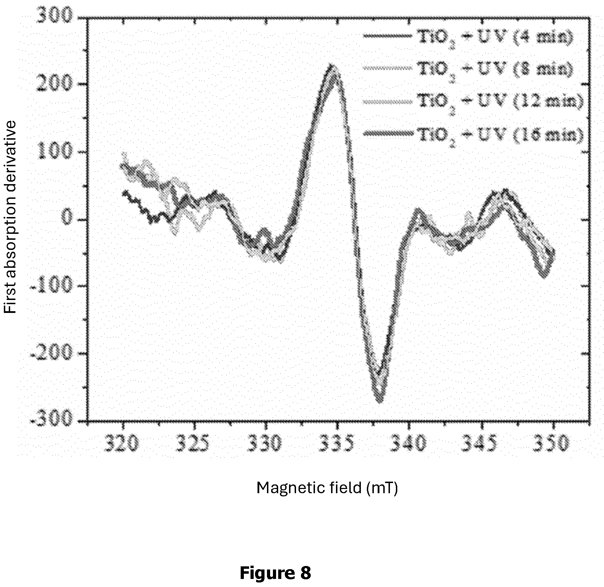

Referring to Figure 8, which shows an Electron Paramagnetic Resonance Spectroscopy (EPR) technique, which is used to determine the formation of free radicals (such as hydroxyl radicals (OH-)) on the surface of materials, the sample to be analyzed is introduced into a quartz tube, which is placed between two coils, which generate a magnetic field that interacts with the sample; in turn, the material is irradiated with microwaves of different frequencies. Microwaves allow unpaired electrons of some chemical species (such as those contained in a radical or ion) to vibrate and be attracted to the poles of the coils (N and S) producing two symmetrical signals, but in opposite directions, as observed in the Figure above.

Also, based on the same Figure, two intense signals can be observed at approximately 334 and 338 mT, which are attributed to unpaired electrons contained in hydroxyl radicals (OH-). This result demonstrates that the UV light (λ = 200 nm) used by the photocatalytic sanitizing device of the present invention allows the formation of these radicals on the surface of TiO2. Furthermore, this result reveals that as the UV light exposure time of TiO2 increases, there is also an increase in the formation of OH- radicals.

### Conditions

The experiment was performed using gravity and impact sedimentation techniques in Petri dishes with trypcasein soy agar medium in Petri dishes of 55 mm and 100 mm diameter. The analysis was performed in an open area without air control in order to clearly see the effect of the device on air by comparing the amount of CFU in ambient air against the result after treatment with the device. If these tests were performed in a laboratory environment, the amount of initial viable particles would be minimal and the effect of the device on air treatment would not be seen. The selected location was an area surrounded by gardens and trees within the university center of exact sciences and engineering (CUCEI).

Before starting the analysis, the surface of the device was cleaned using 70 % ethanol to eliminate possible viable particles that were previously found on the device. Additionally, a 5-minute purge period was performed with the device on to ensure that viable particles that were inside the device before the analysis was performed were not picked up at the start of the analysis.

To determine the state of the ambient air in terms of viable particles, control plates were used that were placed at the air inlet of the device. This serves as a reference to compare the air that entered the device with the air that left it. This test is based on the sedimentation capacity of viable particles by means of gravity to come into contact with the Petri dish.

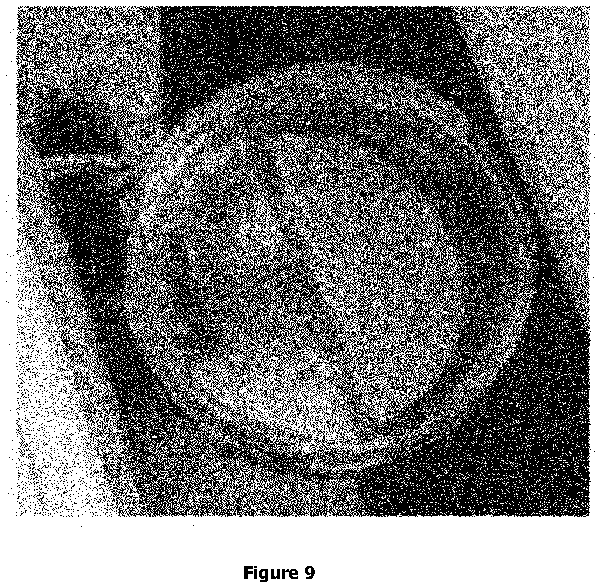

For the analysis test, an impact sampling technique was used, where a box with sterile medium was placed at the air outlet, after the purge period had been completed and with the device still on. Because the air outlet direction is upwards, the box had to be placed inverted with respect to the surface of the device (see Figure 9), in this way the exhaust air came into direct contact with the medium. Because the device is on during the analysis, the air flow leaves the device and comes into contact with the medium by impact.

On the other hand, Figure 10 shows the photocatalytic sanitizing device, in a particular mode, in the area selected for the analysis, where the plates placed at the top for air inlet and at the bottom for air outlet can be observed.

Once the analysis test was set up, the photocatalytic sanitizing device was turned on for a period of 15 minutes at a flow rate of 2 m³ per minute. To analyze the repeatability of the result, the test was performed in triplicate on three different days. Once the test period was over, the plates were removed from the device and incubated at 37°C for a period of 3 days. After the incubation period, the colonies present were counted, and the result was expressed in CFU/plate.

### Results

After the 3-day incubation period, the plates were removed from the incubator; Figure 11 illustrates the plates after the incubation period, where the difference in the number of colonies present between the control plates and the experimental plates can be clearly seen. In particular, and in order to increase the clarity of said Figure:
a) and b) results of test 1 for air inlet and outlet control respectively;
c) result of air entry in test 2;
d) result for air output in test 2; and
e) and f) results in test 3 for air inlet and outlet respectively.

The result of the colony count is shown in the following table 1. For the plates that were placed in the air outlet, no growth was seen after the incubation period with the exception of test 3 where a CFU was found near the edge of the plate. Meanwhile we can see that on the air inlet plates the number of UFCs is counted in dozens. The significant difference in the number of colonies at the air inlet and outlet can be seen. This result is interpreted as the operation of the device having a clear effect on the reduction of CFU of viable particles found in ambient air.

**TABLE 1**

| **Results of colony counting** | | |
|---|---|---|
| Test No. | UFC/Plate | |
| | Inlet | Outlet |
| Test 1 | 21 | 0 |
| Test 2 | 33 | 0 |
| Test 3 | 57 | 1 |

Based on the foregoing, it is clear that the photocatalytic sanitizing device of the present invention generates a significant decrease in the amount of viable particles present in the air, reaching an amount capable of complying with the limit stipulated in the regulations in force in certain sectors of the industry, being that, with added emphasis, said significant decrease is a result of the photocatalytic process used by the photocatalytic sanitizing device and, mainly and particularly, derived from the photocatalytic components used and the mixing ratio thereof, which, as described throughout this document, said innovative, novel and advantageous mixing ratio allows, precisely, that the contaminants/organic material be substantially and successfully removed from the air once the steps of the method of the present invention are carried out.

Many modifications and other embodiments of the invention will come to mind to one skilled in the art to which the invention pertains, having the benefit of the teachings presented in the foregoing descriptions and associated drawings. Therefore, it is to be understood that the invention is not to be limited to the specific embodiments described, but that modifications and other embodiments are intended to be included within the scope of the appended claims. Although specific terms are used here, they are used only in a generic and descriptive sense and not for limiting purposes.

Below is a list of elements with their respective identifiers:
1 photocatalytic sanitizing device
10 outer shell
11 internal volume
12 opening gate
20 inlet and intake assembly
21 fan
30 output and ejection assembly
31 mechanical filter
32 output
33 air quality sensor
41 heat exchanger (Peltier plate)
42 humidity sensor
43 temperature sensor
50 retainer/mixer assembly
61 radiation emitting source
62a and 62b photocatalytic component storage tank
70 decontamination base
81 microcontroller
90 power supply

## Claims

1. A photocatalytic sanitizing device (1), capable of removing/reducing the number of organic matter/contaminants by means of a photocatalytic reaction process; the device comprising: an outer shell (10), wherein the outer shell (10) forms an internal volume (11); an inlet/intake assembly (20); an output/ejection assembly (30); a humidity controller; a retainer/mixer assembly (50); a photocatalyst/photocatalyst assembly; a decontamination base (70); a control unit; and a power supply (90); where the inlet/intake assembly (20) draws a quantity of ambient air from the immediate environment with which the photocatalytic sanitizing device (1) interacts, and conducts it to the interior volume (11); wherein the photocatalytic/photocatalyst assembly is made up of at least one radiation emitting element (61) and at least one storage tank for photocatalytic components (62a, 62b), wherein the radiation emitting element (61) is configured to emit radiation, necessary to carry out the photocatalytic reaction process; wherein the retainer/mixer assembly (50) is made of a porous material or with a density of porosities, and is configured to retain/store the photocatalytic components, coming from and/or supplied by each of the storage tanks (62a, 62b), by means of absorption, as well as a liquid in such a way that a mixture of the liquid and the photocatalytic components is formed in the form of an amalgam; wherein the photocatalytic components are titanium dioxide (TiO2) and zinc oxide (ZnO) and, wherein the mixture of photocatalytic components contained in the retainer/mixer assembly (50) is a mixing ratio of 1:1 or other than 1:1; where, the mixing ratio can adsorb the water present in the ambient air contained in the internal volume (11), allowing to control the level of humidity present therein; wherein the decontamination base (70) is a structure connected to or mechanically interacting with the internal portion of the external casing (10), which is arranged in a portion above the photocatalytic assembly, the decontamination base (70) being configured to receive on its upper surface a certain object in such a way that said object can be decontaminated, supported and/or using the photocatalytic reaction process; wherein the outlet/expulsion assembly (30) further comprises at least one air quality sensor (33), configured to determine and/or check the quantity of contaminants/organic matter present and/or remaining in the air contained in the interior volume (11), such that, after a certain operating time of the device (1), that is, after a time during which the photocatalytic reaction process is carried out, the sensor (33) will send information to the control unit relating to the current air quality, and based on this, determine whether the outlet/expulsion assembly (30) should retain the air contained in the interior volume (11) for a longer exposure time to decontamination based on the photocatalytic process, or on the contrary, release the contained air into the immediate environment, which means that the sensor (33) has detected that the air quality is high and/or optimal and therefore, there is no remaining organic matter/contaminants to be eliminated.

2. The photocatalytic sanitizing device (1) according to claim 1, wherein the mixture of photocatalytic components comprises a mixing ratio of 1:1, 2:1, 3:1, 1:1, 2:1, 3:1, 4:1, 5:1, 6:1, 7:1, 8:1, 9:1, 10:1, 20:1, 50:1 or higher, as well as 1:2, 1:3, 1:4, 1:5, 1:6, 1:7, 1:8, 1:9, 1:10, 1:20, 1:50 or higher.

3. The photocatalytic sanitizing device (1) according to claim 1, wherein the outer casing can be made of any material selected from the group comprising iron, carbon steel, stainless steel, aluminum, alloys, combinations thereof and/or the like, acrylic, PET or PETE type plastics (polyethylene terephthalate, HDPE (high density polyethylene), PVC (polyvinyl chloride), LDPE (low density polyethylene), PP (polypropylene), PS (polystyrene), combinations thereof and/or the like.

4. The photocatalytic sanitizing device (1) according to claim 1, wherein the outer shell (10) can be formed/constituted into any geometric body, such as cylinders, prisms, complex and/or irregular shapes and/or combinations of shapes, wherein the dimensions of the outer shell (10) are directly dependent on the size of the internal volume (11).

5. The photocatalytic sanitizing device (1) according to claim 1, wherein a relatively small object, i.e. whose volume is, for example, 5 cm³, 10 cm³, 15 cm³, 20 cm³, 50 cm³, 100 cm³ or more and/or a relatively large object, i.e. which volume is, for example, cm³, 0.5 m³, 1 m³, 2 m³, 5 m³, 10 m³, 20 m³ or more, can be introduced into the internal volume (11) and arranged on the decontamination base (70).

6. The photocatalytic sanitizing device (1) according to claim 1, wherein the inlet/intake assembly (20) further comprises a fan (21) that, by its operation, draws and directs the ambient air into the internal volume (11).

7. The photocatalytic sanitizing device (1) according to claim 1, wherein the outlet/expulsion assembly (30) comprises: a mechanical filter (31) which is a filter made up of activated carbon pellets and/or any other material with inherent decontaminating and/or purifying properties, which, in addition to offering a certain resistance to the passage of air when trying to leave the internal volume (11), acts as an additional decontamination step, removing/withdrawing the organic matter present in the sanitized air, in case there is a remnant, after the photocatalytic process prior to being released/expelled back into the environment, and an output (32), controlled by the control unit, such that, once the decontamination process is finished and the air quality sensor (33) determines that the air quality is high, the output (32) can switch from a closed state to an open state and thus release the already decontaminated air, wherein the air quality sensor is a sensor selected from the group comprising a GP2Y1014AU dust and/or air quality sensor, a ZH0 laser dust sensor, a HIC192 infrared dust sensor, a QSA2700 dust sensor, a particles per million sensor, combinations thereof and/or the like.

8. The photocatalytic sanitizing device (1) according to claim 1, wherein the humidity controller may optionally comprise a heat exchanger element (41), configured to transmit energy and increase/decrease the temperature of a fraction of the air that is drawn into the internal volume (11), with the intention of condensing a fraction of the humidity present in said air and providing the internal volume (11) with a desired humidity.

9. The photocatalytic sanitizing device (1) according to claim 1, wherein the moisture fraction condensed by the heat exchange element (41) is retained in the retainer/mixer assembly (50) and can be used for the photocatalytic process, as an amalgamating medium and/or can be used as an additional means to control the humidity level within the internal volume (11).

10. The photocatalytic sanitizing device (1) according to claim 1, wherein the humidity controller further comprises at least one humidity level sensor (42), and at least one temperature sensor (43) incorporated in the internal volume (11) and/or distributed in different portions thereof and wherein the humidity level sensor (42) and the temperature sensor (43) are respectively connected to the control unit, wherein the humidity level sensor (42) is configured to detect an inadequate overall and/or average level of humidity within the internal volume (11) and the control unit can increase/decrease the voltage/current flowing through the heat exchanger element (41) in order to increase/decrease the amount of energy transmitted to the air of the internal volume (11), and wherein the temperature sensor (43) is configured to detect changes in the temperature of the air contained in the internal volume (11), feedback said information to the control unit in order to improve the efficiency of energy transfer carried out by the control unit.

11. The photocatalytic sanitizing device (1) according to claim 1, wherein the retainer/mixer assembly (50) is a porous material or one with a density of porosities with the capacity to retain/absorb liquids, granular solids (pulverized and/or in powder form), such as a sponge, foam materials based and/or manufactured from ethylene-vinyl acetate polymers (EVA), polyethylene (PE), combinations thereof and/or the like.

12. The photocatalytic sanitizing device (1) according to claim 1, wherein the liquid contained in the retainer/mixer assembly (50) is water, preferably double-distilled water, wherein the contained liquid can be alternatively arranged on the retainer/mixer assembly (50) and cannot necessarily be provided by the condensation of a fraction of humidity present in the air within the internal volume (11).

13. The photocatalytic sanitizing device (1) according to claim 1, wherein the retainer/mixer assembly (50) may optionally contain, in any other way, the photocatalytic components and not necessarily with the aid of the porous property of the material, being capable of retaining said photocatalytic components in a solid form as a tablet (not shown) that reacts in the same way as the porous material once it receives and/or is irradiated with UV light.

14. The photocatalytic sanitizing device (1) according to claim 1, wherein the radiation emitting source (61) of the photocatalytic/photocatalyst assembly can be any means capable of generating electromagnetic waves, such as germicidal lamps, wide-frequency ultraviolet radiation lamps ranging from 100 to 200 nm, magnetrons and/or controlled microwave emission means, combinations thereof, and/or the like.

15. The photocatalytic sanitizing device (1) according to claim 1, wherein both photocatalytic components can be directly or previously arranged within the retainer/mixer assembly (50), complying with a 9:1 mixing ratio.

16. The photocatalytic sanitizing device (1) according to claim 1, wherein the decontamination base (70) is made of the same material as the outer shell (10) and its dimensions are directly related to the shape and dimensions adopted by said shell (10), where the decontamination base (70) further comprises grooves passed through its surface, so that the hydroxyl radicals formed in the photocatalytic process can circulate through the base.

17. The photocatalytic sanitizing device (1) according to claim 1, wherein the control unit comprises at least one and up to "n" number of microcontrollers (81) connected to a plurality of sensors and components of the device (1), wherein the microcontroller (81) is connected to the radiation emitting source (61), whereby, it can start and/or conclude a cycle of the photocatalytic process depending on the exposure time of the air/object to the hydroxyl radicals and/or depending on the air quality detected by the air quality sensor (33).

18. The photocatalytic sanitizing device (1) according to claim 1, wherein the power supply (90) is at least one and up to "n" number of power supplies and is any of the group comprising commercially known direct current and alternating current power supplies, a 12v, 10a 120w, 110vac, 220vac switched power supply, a 12v or 5v mini switched power supply, combinations thereof and/or the like.

19. The photocatalytic sanitizing device (1) according to claim 3, wherein furthermore, an additional treatment may be applied to the material of the outer shell (10); such as a coloring treatment, in case of using acrylics/plastics, in order to achieve an opaque color or screen to dissipate/reduce the amount of radiation generated by the radiation emitter (61), and/or coatings to increase resistance to corrosion and/or damage inherent to the weather, in case of using metallic materials.

20. The photocatalytic sanitizing device (1) according to claim 3, wherein the outer shell (10) further comprises an openable door (12), which in an open configuration, allows the determined object to be placed on the decontamination base (70) and, in a closed configuration, ensures the tightness and hermeticity of the internal volume (11) of the photocatalytic sanitizing device (1).

21. The photocatalytic sanitizing device (1) according to claim 4, wherein the internal volume (11) has a capacity ranging from 50 cm³ to "n" cm³ or from 25 cm³ to "n" cm³ or from 5 cm³ to "n" cm³.

22. The photocatalytic sanitizing device (1) according to claim 6, wherein the fan (21) can be made of any material selected from the group comprising iron, carbon steel, stainless steel, aluminum, alloys, combinations thereof and/or the like, PET or PETE type plastics (polyethylene terephthalate, HDPE (high density polyethylene), PVC (polyvinyl chloride), LDPE (low density polyethylene), PP (polypropylene), PS (polystyrene), combinations thereof and/or the like and wherein the fan (21) is capable of displacing/dragging a volumetric flow ranging from 0.5 kg/m³ to "n" kg/m³.

23. The photocatalytic sanitizing device (1) according to claim 8, wherein the heat exchange element (41) is at least one and up to "n" number of heat exchange components distributed in different portions of the internal volume (11).

24. The photocatalytic sanitizing device (1) according to claim 8, wherein the heat exchange element (41) is an electrothermal device, such as a resistor assembly/bank (Joule effect) and/or a 12706-type thermoelectric Peltier Plate/Cell Cooler 12v 5A 60W, and/or combinations thereof.

25. The photocatalytic sanitizing device (1) according to claim 10, wherein the humidity and temperature sensor (42, 43) may be combined/integral sensors such as Dht21/am2301, Dht11, Sht30, SHT31-ARP-B temperature and humidity sensors, combinations thereof and/or the like.

26. The photocatalytic sanitizing device (1) according to claim 11, wherein the retainer/mixer assembly (50) stores/retains a quantity of photocatalytic components ranging from 0.5g to "n" number of grams and up to 0.5ml, and up to "n" number of milliliters, depending on the type of presentation of both elements.

27. The photocatalytic sanitizing device (1) according to claim 12, wherein the liquid is intended to contain and mix the photocatalytic components (to amalgamate both components (TiO2 and ZnO), avoiding the formation of lumps, which consequently results in an improvement in the efficiency of the photocatalytic process.

28. The photocatalytic sanitizing device (1) according to claim 15, wherein the storage tanks (62a, 62b) can be dispensed with.

29. The photocatalytic sanitizing device (1) according to claim 17, wherein the microcontroller (81) is connected to the humidity controller, in order to control the operating time and/or energy supplied to the heat exchanger element (41) and thereby control the level of humidity present in the interior volume (11) of the photocatalytic sanitizing device (1), wherein the microcontroller (81) is further connected to the storage tanks (62a, 62b) of the photocatalytic components, to coordinate the precise and controlled release of each photocatalytic component, ensuring that a desired mixing ratio, such as 9:1, is deposited in the retainer/mixer assembly (50).

30. The photocatalytic sanitizing device (1) according to claim 17, wherein the at least one or the plurality of microcontrollers (81) is any selected from the group comprising AMCC, Altera, Analog Devices., Atmel, Charmed Labs, Cypress MicroSystems, Dallas Semiconductor, ELAN Microelectronics Corp, Freescale Semiconductor, Fujitsu, Holtek, Infineon, Intel, Lattice Semiconductor, Microchip Technology, National Semiconductor, combinations thereof and/or the like.

31. A method for decontaminating, sanitizing, or reducing the amount of organic matter present in ambient air, particularly relying on an improved photocatalytic process, and using the photocatalytic sanitizing device (1) of any of claims 1 to 18; the method comprising: suctioning contaminated air from the immediate environment, i.e. close to the photocatalytic sanitizing device (1), and conducting/directing it into the interior of the photocatalytic sanitizing device (1) by means of actuating a fan (21); ensuring a controlled humidity level, using the humidity controller and, in particular, relying on the improved overall hygroscopic property derived from the synergy of the photocatalytic components and the 9:1 mixing ratio; activate the radiation emitting source (61) and, consequently, irradiate and/or supply/direct the radiation emitted by it to the retainer/mixer assembly (50), thus starting the photocatalytic reaction process; eliminate any organic matter present in the internal volume (11) of the photocatalytic sanitizing device (1), once the hydroxyl radicals (OH), generated during the enhanced photocatalytic process come into contact with them; assessing the quality of the air within the internal volume (11) of the photocatalytic sanitizing device (1), using the plurality of sensors and after the air within the internal volume (11) has been exposed to the hydroxyl radicals resulting from step iii); such that, if the plurality of sensors determine that the quality of the air contained in the internal volume (11) is at a low and/or unsatisfactory level, that is, that considerable amounts of contaminants/organic matter still remain, the radiation emitting source (61) will continue to be on, causing the photocatalytic reaction to continue, and hydroxyl radicals to be generated uninterruptedly; and expel the air (now sanitized/decontaminated) from the internal volume (11) and direct it back to the immediate environment.

32. The method according to claim 19, wherein the photocatalytic sanitizing device (1), according to any of claims 1 to 18, is used to carry it out.

33. The method according to claim 19, wherein the ambient air may be air close to the photocatalytic sanitizing device (1) within an enclosed space such as a room and/or determined area, and wherein said air is conducted from the inlet/intake assembly (20) and directed and contained in the internal volume (11) of the photocatalytic sanitizing device (1).

34. The method according to claim 19, wherein the level is controlled so as to ensure sufficient humidity to increase the efficiency of the photocatalytic reaction and thereby increase the sanitization efficiency.

35. The method according to claim 19, wherein the photocatalytic reaction process starts when the radiation emitting source (61) emits and supplies radiation on the retainer/mixer assembly (50), which contains the photocatalytic components in the 9:1 ratio, generating hydroxyl radicals (OH), which will be released and spread (based on particle dynamics) within the internal volume (11) of the photocatalytic sanitizing device (1), wherein the organic matter present in the internal volume (11) may be contaminated air introduced in step i) and, in addition, may be the organic matter present on a plurality of surfaces of a given object that has been placed in the internal volume (11), particularly, deposited on the decontamination base (70), wherein the contaminated air within the internal volume (11) and which comes into contact with the hydroxyl radicals formed in step iii) is in continuous movement, and said air allows the hydroxyl radicals to be conducted/directed until they come into contact with the plurality of surfaces of the determined object arranged in the internal volume (11), thus achieving that the contaminants in the determined object are reduced/eliminated.

36. The method according to claim 19, wherein the plurality of sensors can carry out tracking and/or determinations of the quality of the air contained in the internal volume (11) in different periods of time.

37. The method according to claim 19, wherein the outlet/expulsion assembly (30), comprising an opening/outlet (32), switches to an open state so that the air, already sanitized/decontaminated, is directed out of the internal volume (11) towards the environment.

38. The method according to claim 19, wherein prior to coming into contact and/or being returned to the environment, the air (already sanitized/decontaminated) passes through a mechanical filter (31) in order to retain any remaining contaminant.

39. The method according to claim 19, wherein once the air (sanitized/decontaminated from step vi) is expelled from the internal volume (11) of the photocatalytic sanitizing device (1), steps i) to vi) previously referred to are repeated again, uninterruptedly and/or cyclically such that the photocatalytic sanitizing device can eliminate organic matter/contaminants gradually, taking fractions of contaminated air from the immediate environment and until the same (contaminated air), in a certain operating time, is completely decontaminated, that is, there are no remnants of organic matter in the room/area of interest.
